**Europäisches Patentamt**

**European Patent Office**

**Office européen des brevets**

(11) Numéro de publication : **0 317 507 B1**

(12) **FASCICULE DE BREVET EUROPEEN**

(45) Date de publication du fascicule du brevet :
08.04.92 Bulletin 92/15

(51) Int. Cl.⁵ : **G10K 9/10, G10K 11/28**

(21) Numéro de dépôt : **88810758.8**

(22) Date de dépôt : **07.11.88**

(54) **Dispositif pour agir par vibrations ultrasonores sur un objet.**

(30) Priorité : **19.11.87 CH 4473/87**

(43) Date de publication de la demande :
**24.05.89 Bulletin 89/21**

(45) Mention de la délivrance du brevet :
**08.04.92 Bulletin 92/15**

(84) Etats contractants désignés :
**AT BE CH DE ES FR GB GR IT LI LU NL SE**

(56) Documents cités :
**DE-C- 278 700**
**FR-A- 455 868**
**US-A- 4 095 667**
**US-A- 4 589 415**
**US-A- 4 692 139**

(73) Titulaire : **Ferton Holding**
**rue de l'Hôpital**
**CH-2800 Delemont (CH)**

(72) Inventeur : **Favre, Robert**
**5 chemin du Levant**
**CH-1005 Lausanne (CH)**

(74) Mandataire : **Gauger, Hans-Peter, Dipl.-Ing. et al**
**Müller, Schupfner & Gauger**
**Maximilianstrasse 6 Postfach 10 11 61**
**W-8000 München 1 (DE)**

EP 0 317 507 B1

## Description

On connaît divers genres de générateurs d'ondes de choc ultrasonores opérant notamment par décharges électriques, effet piézo-électrique, ou détonations d'explosifs chimiques. Mais leur prix de revient est élevé et ils sont mal adaptés à certaines applications, notamment aux lithotriteurs utilisés en médecine pour la destruction de pierres rénales.

L'idée de base du brevet US 4 589 415 est de réaliser un lithotriteur opérant par ondes de choc, transmises ponctuellement à travers les tissus vivants par un faisceau de sondes intermédiaires - sans contact avec la pierre rénale à détruire - de manière à ce que la densité d'énergie injectée par chaque sonde soit assez faible pour ne pas endommager les tissus, tandis que la somme des énergies concentrées sur la pierre doit être assez élevée pour la détruire. La possibilité de n'utiliser qu'une sonde n'est donc que théorique, d'autant plus que l'inévitable divergence des ondes, dès la sortie de la sonde, implique une énergie supplémentaire. Les principaux inconvénients de cette technique découlent de l'expérience acquise avec les lithotriteurs extracorporels connus. On sait par exemple que pour être efficace la concentration des ondes de choc sur la pierre doit se réaliser dans un volume de quelques mm³, ce qui n'est imaginable ici qu'à raison de deux dispositifs de visualisation très onéreux, formant un certain angle, pour permettre l'orientation exacte des sondes. On sait également que pour être efficaces toutes les composantes de l'onde de choc doivent arriver "en phase" au foyer, ce qui implique ici, pour le moins, un tâtonnement très long et casuel. On sait encore que si la pierre n'est pas retenue par les tissus adjacents, elle se déplacera sous l'effet des ondes de choc, d'où la nécessité d'adapter en permanence l'orientation des sondes avec toutes les difficultés que cela implique. Le procédé connaît enfin les mêmes limites que la lithotritie extracorporelle, à savoir que la pierre n'est plus accessible lorsqu'elle se trouve dans l'uretère, derrière l'ossature pelvienne.

Le brevet allemand 278 700 (de 1913) décrit un mécanisme pour mettre et maintenir en vibration une membrane d'un avertisseur acoustique, au moyen d'un mécanisme transformant le mouvement de rotation d'un disque entraîné par un moteur, en un mouvement alternatif de translation d'une pièce solidaire de la membrane, grâce à une bille disposée entre cette pièce et ce disque, dans des alvéoles de ces deux organes. Dans cette construction, il n'y a pas de production d'ondes de choc, mais simplement production d'un signal sonore.

Le brevet français 455 868 décrit aussi un avertisseur acoustique mécanique à membrane vibrante, comportant un piston prévu pour effectuer un mouvement de va-et-vient dans un cylindre sous la commande de moyens pneumatiques. Ce piston vient repousser périodiquement le centre de la membrane, occupé par une enclume ad hoc, pour entretenir sa vibration. Non seulement la production d'ondes de choc ultrasonores serait inutile pour un avertisseur, mais cette éventualité est exclue, l'impédance acoustique de la membrane étant adaptée à celle de l'air, qui est 70'000 fois plus faible que celle du piston (rappelons que l'impédance acoustique d'un milieu homogène est égale au produit de sa masse spécifique par la vitesse du son dans ce milieu).

La présente invention vise à fournir un dispositif pour agir par vibrations ultrasonores sur un objet tel qu'une pierre rénale qu'il s'agit de fragmenter pour permettre son élimination naturelle, qui soit très efficace et utilise dans ce but des ondes de choc ultrasonores et qui soit de construction simple et peu coûteuse. Elle a pour objet un dispositif qui est conforme à la revendication 1.

Le dessin annexé représente, à titre d'exemples, trois formes d'exécution du dispositif selon l'invention.

Figure 1 est une vue schématique, en coupe axiale, de la première forme d'exécution, prévue pour agir comme lithotriteur.

Figure 2 est une vue partielle, en coupe axiale, de la seconde forme d'exécution, prévue pour agir comme lithotriteur extracorporel non invasif.

Figure 3 est une vue partielle, en coupe axiale, de la troisième forme d'exécution, prévue aussi pour agir comme lithotriteur extracorporel non invasif.

Figure 4 est une vue de détail, en coupe axiale, du projectile utilisé dans la troisième forme d'exécution.

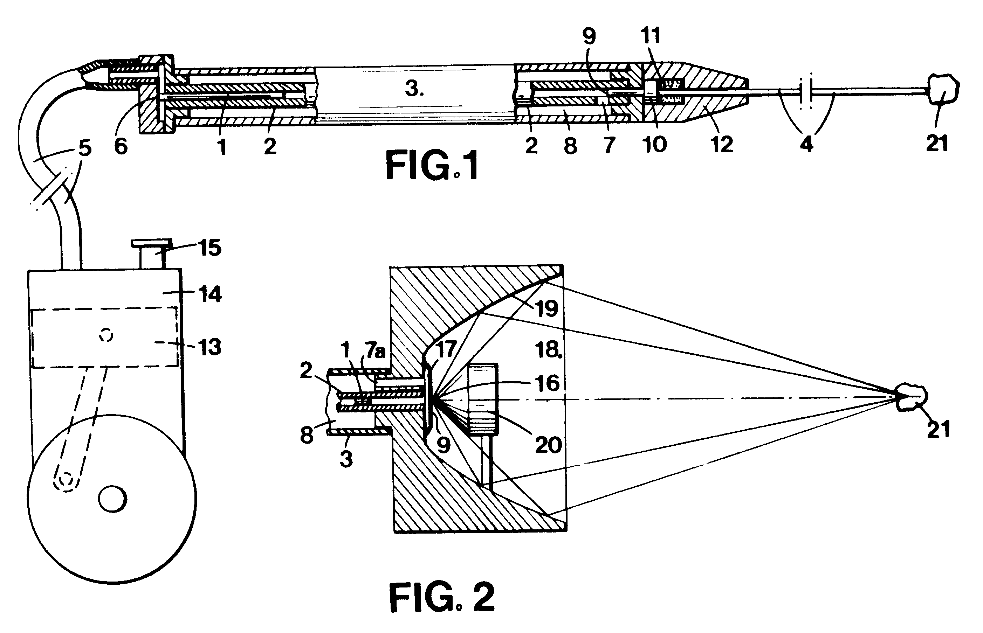

Sur la figure 1, un projectile filiforme 1, en acier, est disposé à l'intérieur d'un tube 2 formant sarbacane, pour glisser et osciller à son intérieur, entre une position ou il est en contact avec une butée de départ 6 et une interface 9 d'entrée d'un guide d'onde 4, également filiforme et en acier.

Le mouvement d'oscillation est imparti au projectile 1 par un compresseur 14 à piston 13, qui est de préférence incorporé dans le même corps que le reste du dispositif. Lors de la course de travail (vers le haut sur la fig. 1) du piston 13, de l'air comprimé est envoyé dans un tube flexible 5 reliant la pompe à la sarbacane 2 et chasse le projectile 1 vers l'interface 9, qu'il vient violemment percuter à la fin de sa course, ce qui produit une onde de choc se transmettant au guide d'onde 4. Celui-ci est monté glissant dans un support 12. Il est retenu axialement par un anneau 10 qui en est solidaire et dont la masse associée peut servir d'adaptateur d'impédance entre le projectile et le guide d'onde. 11 est une butée amortisseur pare-choc.

Lors de la course de retour du piston 13 du compresseur (vers le bas sur la fig. 1), il se crée une dépression dans le tube 5, ce qui fait revenir le projectile 1 dans sa position de départ ou il est en contact avec la butée 6. Ainsi, un mouvement alternatif est imparti au projectile 1 à l'intérieur de la sarbacane. Sa fréquence pourra être, par exemple, de 25 ou 30 oscillations doubles par seconde, correspondant à un compresseur entraîné directement par un moteur électrique à quatre pôles, pour une fréquence du courant d'alimentation de 50 à 60 périodes par seconde. Dans ce cas, la pression sera avantageusement maintenue très basse, notamment pour réduire l'échauffement.

Le tube sarbacane 2 est disposé axialement dans une enveloppe cylindrique coaxiale 3 laissant entre lui et cette enveloppe une chambre annulaire 8 reliée à l'intérieur de la sarbacane par une fenêtre 7 traversant la paroi de cette sarbacane. La chambre annulaire 8 forme un réservoir auxiliaire, alimenté à partir du compresseur par les fuites autour du projectile 1, à une pression dérivée de la moyenne pondérée des pressions en amont de la sarbacane, permettant le rappel du projectile, le moment venu, tout en assurant l'étanchéité de la sarbacane par rapport aux poussières atmosphériques ambiantes ou à un liquide éventuel de stérilisation.

Le choc violent du projectile 1 contre le guide d'onde 4 engendre une onde de choc à l'entrée du guide d'onde 4, dont la durée est égale au temps qui lui est nécessaire pour parcourir deux fois la longueur du projectile 1, à la vitesse d'environ 5000 m/sec, soit approximativement 10 us pour un projectile de 25 mm de longueur.

Pour que l'onde de choc se traduise par une élongation maximale du guide d'onde, la longueur de ce dernier doit, par conséquent, être au moins deux fois plus élevée que celle dudit projectile.

Les pressions extrêmes transmises à la sarbacane sont réglables par la soupape d'admission (non représentée) du compresseur, et/ou par une soupape 15 limitant la surpression; ceci permet d'ajuster la quantité d'air dans le circuit de propulsion et, de ce fait, l'amplitude des ondes de choc.

La présence du réservoir auxiliaire 8 permet, dans une autre variante, le remplacement du compresseur 14 par une alimentation séquentielle de la sarbacane, par l'intermédiaire d'une valve pneumatique, à partir d'une source d'air comprimé usuelle, de pression et de débit appropriés.

Lorsque le dispositif décrit est utilisé comme lithotriteur à percussion, le guide d'onde 4 est amené au contact de la pierre rénale 21, par la voie naturelle, à travers un rénoscope. Ce guide d'onde transmet à la pierre 21, par son extrémité libre, les ondes de choc produites par la percussion du projectile 1 contre l'interface 9. Ainsi, cette extrémité libre du guide d'onde 4 agit directement sur la pierre en lui transmettant les ondes de choc ultrasonores qu'il reçoit, en opérant par burinage de contact de la pierre à détruire.

On a indiqué plus haut la possibilité de travailler à environ 25 ou 30 percussions par seconde. Dans ce cas, le projectile 1 pourra avoir 8 mm de long et 4 mm de diamètre, tandis que le guide d'onde 4 peut avoir 1 mm de diamètre seulement et 500 mm de long, lorsqu'il s'agit d'atteindre la pierre à travers un rénoscope.

Le dispositif selon fig. 1 peut aussi être utilisé, dans une variante, comme lithotriteur opérant par voie transcutanée à travers un néphroscope. Dans ce cas, le guide d'onde 4 pourra avantageusement être tubulaire et agencé de manière à permettre une circulation d'eau de lavage.

Dans le cas où le compresseur 14 est remplacé par une source d'air comprimé à pression constante, de par exemple 3 atmosphères, il est évident qu'une valve pneumatique à trois voies peut être prévue pour commuter la sarbacane 2, alternativement sur cette pression, respectivement sur l'atmosphère, par séquences de, par exemple, 25 ms, respectivement 60 ms. L'intensité des chocs produits est réglable dans ce cas par divers moyens connus de l'homme du métier, par exemple un manodétendeur sur ladite source d'air comprimé.

Le dispositif selon fig. 1 ainsi que ses variantes sont applicables à diverses opérations mécaniques pour le travail d'un objet, par exemple pour sculpter ou graver des matériaux, notamment pierreux.

La forme d'exécution du dispositif représentée sur la figure 2 est utilisable comme lithotriteur extracorporel, donc non invasif, les ondes de choc se propageant vers la pierre rénale à détruire en passant à travers le milieu biologique après avoir traversé un liquide remplissant le guide d'onde comme on va l'expliquer.

Sur la figure 2, on n'a pas représenté le compresseur et la partie de gauche de la sarbacane 2 et de l'enveloppe 3 de la figure 1, car elle est identique à ce que montre cette figure 1.

On voit en figure 2, comme sur la figure 1: le projectile 1, ici très court pour s'adapter à la longueur d'onde exigée, la sarbacane 2 reliée au réservoir auxiliaire 8 par une fenêtre 7a, et l'enveloppe cylindrique extérieure 3.

Par contre, le guide d'onde est très différent de celui de la fig. 1. L'interface d'entrée du guide d'onde est formée ici par une membrane d'étanchéité 17 de très haute résistance mécanique, dont le centre 16 est disposé pour être percuté périodiquement par le projectile 1 lors de son mouvement oscillant. C'est cette percussion qui produit les ondes de choc qui seront transmises à la pierre 21 à détruire, comme on va le décrire maintenant.

Le centre 16 de la membrane 17 se trouve à l'un des foyers d'un réflecteur ellipsoïdal 19 dont l'autre foyer, 21, est intracorporel, étant amené (par observation sur un écran de radioscopie par exemple) à se trouver à l'intérieur de la pierre rénale à détruire. L'espace compris entre le réflecteur 19 et le corps du patient est rempli d'un liquide non représenté assurant la transmission de l'énergie cinétique des ondes de choc du réflecteur 19 à la pierre à détruire et la continuité de l'impédance acoustique.

Le guide d'onde comprend un déflecteur 20, de forme conique, coaxial avec la ligne joignant les deux foyers 16 et 21 du réflecteur ellipsoïdal 19. Ce déflecteur a pour fonction de guider vers la surface du réflecteur 19, les ondes de choc qui, en son absence, échapperaient à la focalisation par le réflecteur 19 et par conséquent ne seraient pas concentrées en 21.

Comme dans le cas de la fig. 1, on voit que la course du projectile 1 est considérablement plus grande que son diamètre.

Pour avoir une bonne transmission de l'énergie du projectile au guide d'onde (en évitant une réflexion indésirable), il est nécessaire que le projectile soit de petit diamètre, en pratique de l'ordre de 2 à 5 mm. Si, avec de tels diamètres, on veut obtenir des ondes de choc d'énergie suffisante, avec une pression d'air comprimé ne dépassant pas 3 atmosphères par exemple, il est nécessaire de donner au projectile une course considérablement plus grande que le diamètre de ce projectile, soit par exemple de 100 à 150 mm.

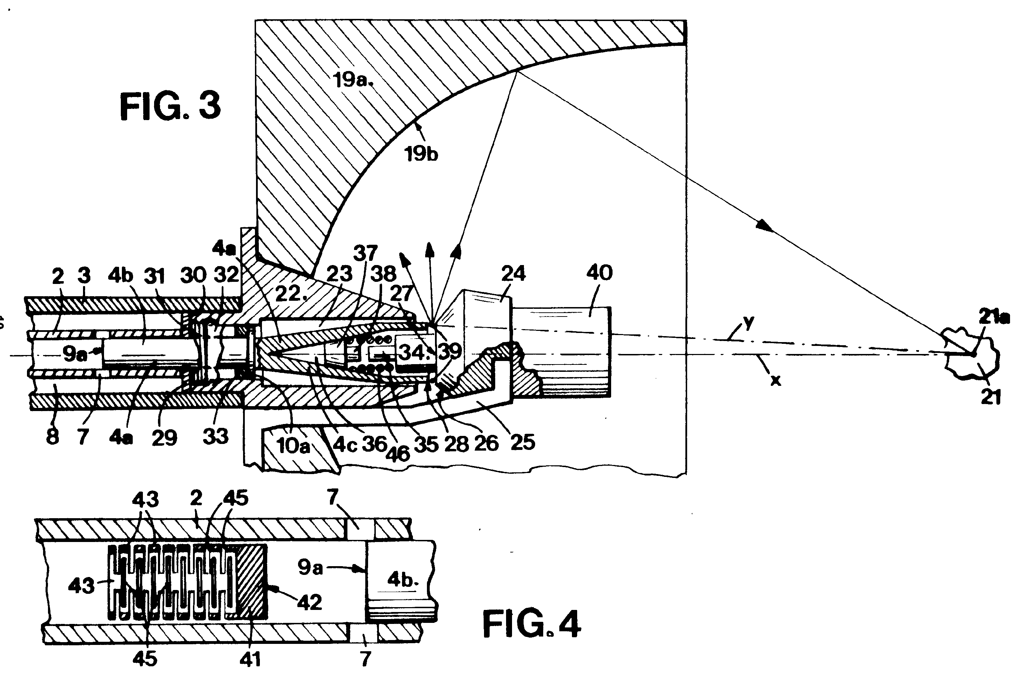

On va décrire maintenant la forme d'exécution selon fig. 3.

La partie de gauche de la fig. 3 montre l'enveloppe 3 et le tube 2 formant sarbacane et dans lequel peut glisser et osciller un projectile non représenté. On voit en 7 des trous traversant le tube 2, pour faire communiquer la chambre annulaire 8 avec l'intérieur du tube 2, comme dans le cas de la fig. 1. Dans ce cas, le diamètre intérieur du tube 2, et par conséquent aussi le diamètre du projectile, sont notablement plus grands que dans le cas de la fig. 1. Ils peuvent être, par exemple, de 4 à 8 mm.

Mais ici le guide d'onde comprend une pièce 4a présentant une partie cylindrique 4b se terminant à droite par une partie évasée en entonnoir 4c. La section transversale de la pièce 4a est constante sur toute sa longueur, pour assurer une impédance acoustique égale sur toute cette longueur, à l'exception négligeable d'une collerette de butée 10a dont on expliquera le rôle plus loin.

Le guide d'onde comprend aussi, comme dans l'exemple selon fig. 2, un réflecteur 19a, dont la surface réfléchissante 19b est celle d'une surface de révolution dérivant, comme il sera expliqué dans la suite, de celle d'un ellipsoïde.

Le réflecteur 19a est coaxial avec l'axe X de la pièce 4a. Il est supporté par une pièce 22 sur laquelle est emmanchée l'enveloppe 3 et qui présente un évidement 23 dans lequel s'étend la partie évasée 4c de la pièce 4.

Le guide d'onde comporte encore une masse réactive 24, coaxiale avec X et supportée par des bras 25 dont un seul est représenté au dessin. Cette masse 24 présente une surface conique 26 et une surface annulaire 27 disposée en regard de l'extrémité ouverte 28 de la partie évasée 4c. La surface annulaire 27 et l'extrémité annulaire 28 forment deux mâchoires circulaires coaxiales, distantes l'une de l'autre de moins d'une longueur d'onde mesurée dans un liquide non représenté qui remplit le volume délimité par 19b. Cette distance peut être constante ou aller en s'agrandissant légèrement vers l'extérieur.

Le tube sarbacane 2 appuie sur une rondelle souple 29 prenant elle-même appui sur l'extrémité gauche 30 de la pièce 22. Cette pièce présente un filetage intérieur dans lequel est vissée une partie filetée 31 d'une pièce 32 traversée axialement par la partie 4b. Cette pièce 32 est prévue pour servir d'appui à un anneau amortisseur pare-choc 33 disposé entre cette pièce 32 et la collerette annulaire 10a déjà mentionnée.

La masse réactive 24 se prolonge vers la gauche sur la fig. 3, par une partie cylindrique, 34, coaxiale avec X et se prolongeant elle-même par une partie cylindrique 35 coaxiale, de plus petit diamètre.

A l'intérieur de la partie creuse, de forme conique, 36, de l'évasement 4c, il est disposé une pièce conique 37 présentant, de son côté faisant face à la partie 35, une partie semblable 38.

Un ressort hélicoïdal de compression 46 est disposé autour de 35 et 38 et prend appui, d'une part sur la partie 34 de la masse réactive 24, et d'autre part sur la pièce conique 37. Ce ressort sollicite donc la collerette 10a à s'appuyer sur l'anneau amortisseur 33.

La surface terminale de gauche, 9a, de la pièce 4a constitue l'interface d'entrée du guide d'onde. C'est elle que vient frapper le projectile (non représenté) effectuant un rapide mouvement de va-et-vient dans le tube sarbacane 2.

Les ondes de choc produites par la percussion de ce projectile contre l'interface d'entrée 9 du guide d'onde se transmettent à l'extrémité de droite 28 de la partie évasée 4c. De là, elles sont transmises, par compression, au liquide dans lequel baigne le guide d'onde, plus exactement dans la partie de ce liquide se trouvant entre les mâchoires annulaires 27 et 28, où elles se concentrent sur un cercle 39 concentrique à X et qui constitue un foyer à partir duquel les ondes de choc ultrasonores rayonnent vers l'extérieur comme on l'a schématisé par des flèches. Les points formant ce cercle se trouvent légèrement en retrait du bord extérieur des mâchoires 27, 28.

La forme géométrique de la surface réfléchissante 19b du réflecteur est définie de la façon suivante: Soit une ligne droite Y passant par un point 21a situé à l'intérieur de la pierre 21 à éliminer et par un point (représenté en 39 sur la fig. 3) du cercle où se concentrent les ondes de choc entre 27 et 28. Cet axe Y est celui d'une ellipse dont les foyers sont en 21a et au point 39. La ligne 19b sur cette fig. 3 est donc une portion d'ellipse. Supposons que l'on fasse tourner l'axe Y de cette ellipse autour de l'axe X, en astreignant cet axe Y à passer toujours au point 21, et à suivre le cercle formé par le lieu géométrique des points 39. La surface décrite par l'ellipse sera celle du réflecteur 19a.

Ainsi donc, les ondes de choc se concentrent, comme on vient de le voir, sur un cercle qui est le lieu géométrique d'un des foyers de l'ellipse génératrice, l'autre foyer étant 21a. Il résulte de cette disposition que les ondes de choc rayonnant vers l'extérieur à partir du cercle formé par les points 39 vont frapper la surface 19b du réflecteur 19a, qui les réfléchira toutes au second foyer 21, où elles se concentreront et provoqueront la destruction de la pierre 21. L'angle solide de sommet 21a formé par ces ondes réfléchies est grand, si bien qu'il n'y a pas de concentration dangereuse dans le voisinage de la pierre 21, même si l'énergie transmise en 21a est grande.

La surface conique 26 de la masse réactive 24 sert de déflecteur, pour obliger celles des ondes émises en 29 qui, en l'absence de ce déflecteur, n'atteindraient pas le réflecteur 19a, à venir se réfléchir sur celui-ci et à se concentrer en 21a.

La position du foyer 21a, intracorporel, sur l'axe X peut être réglée au moyen d'une sonde de positionnement 40, supportée par la masse 24, située sur l'axe X, partant, à l'abri des ondes de choc.

Dans une variante, la pièce 4a pourrait être cylindrique (hormis la collerette 10a) jusqu'aux mâchoires 27, 28. On aurait alors aussi concentration des ondes de choc selon un foyer circulaire formé de points tels que 39 sur la fig. 3.

La durée des chocs pour la lithotritie extracorporelle ne dépassant pas l'ordre de 1 us, la longueur utile du projectile, d'environ 2,5 mm, est nettement inférieure à son diamètre, d'où nécessité de le guider. La fig. 4 représente, en coupe axiale agrandie, un exemple d'un tel projectile. Il comporte un corps cylindrique 41, se prolongeant du côté opposé à sa face 42 venant frapper l'interface d'entrée 9a du guide d'onde dont on voit la partie 4b, par une jupe annulaire mince 43, entaillée en 44 et 45 de façon à former ressort. La masse de cette jupe est très faible, pour éviter que la périphérie de la surface 42 agisse localement de façon exagérément forte sur 9a. Grâce aux entailles 43 et 44, l'effet de la masse de la jupe est atténué, aussi bien lors du choc contre 9a qu'à l'autre extrémité de la course du projectile.

Il est à relever que dans toutes les formes d'exécution représentées au dessin, la course du projectile produisant les ondes de choc par effet balistique est considérablement plus grande que le diamètre de ce projectile. Cette condition est nécessaire pour les raisons suivantes.

Etant donné que c'est la percussion du projectile sur l'interface d'entrée du guide d'onde qui produit les ondes de choc, il s'agit d'imprimer au projectile, par la pression du gaz venant du compresseur ou d'un réservoir, une vitesse à fin de course, telle que la percussion désirée soit obtenue. Une course très petite conduit à l'emploi de pressions de gaz élevées, ce qui a plusieurs inconvénients: dégagement excessif de chaleur, gaspillage d'énergie, complications au niveau du compresseur et de son moteur d'entraînement. En utilisant une course relativement longue, on élimine tous ces inconvénients.

En ce qui concerne le diamètre du projectile, si l'on veut éviter des réflexions indésirables, il ne peut pas être beaucoup plus grand que celui du guide d'onde (par exemple 4 fois dans le cas de la fig. 1, et égalité dans le cas de la fig. 3, par rapport à la partie du guide d'onde située en amont du réflecteur).

Il est clair que le champ médical d'application des dispositifs selon fig. 2 et 3 n'est pas limité à la destruction de pierres rénales. Ces dispositifs sont utilisables aussi pour la destruction non invasive d'autres corps nuisibles pouvant se trouver dans un organisme vivant, tels que certaines tumeurs.

## Revendications

1. Dispositif pour agir par vibrations ultrasonores sur un objet comprenant un générateur d'ondes de choc ultrasonores (1, 2, 3) caractérisé en ce qu'il comporte d'une part, un projectile de forme allongée placé dans un tube formant sarbacane (2), pour y glisser, et des moyens pneumatiques (5) disposés à une extrémité de la sarbacane (2), pour impartir à ce projectile (1) un mouvement de va-et-vient dans la sarbacane (2), d'amplitude considérablement plus grande que la dimension transversale du projectile, et d'autre part, un guide d'onde (4, 19) présentant une interface d'entrée (9) située à l'autre extrémité de la sarbacane (2), et prévue pour être percutée périodiquement par le projectile (1) lors de son mouvement alternatif et générer ainsi par effet balistique des ondes de choc ultrasonores, ce guide d'onde (4, 19) étant agencé pour transmettre ces ondes de choc à leur lieu d'utilisation (21).

2. Dispositif selon la revendication 1, caractérisé par le fait que la partie amont de ladite sarbacane (2), par rapport audit projectile (1), est soumise directement à la pression cyclique d'un cylindre de compresseur, ledit projectile circulant dans le sens aller pendant la phase de haute pression et dans le sens retour pendant la phase de basse pression.

3. Dispositif selon la revendication 1 ou 2, caractérisé par le fait que ladite sarbacane est reliée pneumatiquement, vers son extrémité aval, à un réservoir auxiliaire (8), pour que de l'air accumulé dans celui-ci assure le retour dudit projectile (1) vers sa position de départ.

4. Dispositif selon la revendication 2 ou la revendication 3, caractérisé par le fait que ledit compresseur comporte des moyens réglables pour permettre d'ajuster la quantité d'air dans le circuit de propulsion et, en conséquence, l'amplitude des ondes de choc.

5. Dispositif selon la revendication 1, caractérisé par le fait que le projectile (1) est propulsé par une alimentation séquentielle de la sarbacane (2) à partir d'une source d'air comprimé à pression pratiquement constante.

6. Dispositif selon la revendication 1 et prévu pour être utilisé comme lithotriteur, caractérisé en ce que le guide d'onde est une tige (4), de diamètre et longueur compatibles avec son passage à travers un appareil du type rénoscope et opérant sur la pierre par burinage de contact.

7. Dispositif selon la revendication 1 et destiné à être utilisé pour la destruction, par voie non invasive, d'objets nuisibles, situés dans un organisme vivant, caractérisé en ce que le guide d'onde comporte un réflecteur ellipsoïdal (19), à un foyer extracorporel (16) duquel le projectile (1) vient percuter la surface d'un liquide (18), à travers une fine membrane d'étanchéité à haute résistance (17), pour y produire des ondes de choc, l'autre foyer du réflecteur (19) étant amenable à l'endroit où se trouve l'objet à détruire, le liquide (18) assurant la continuité de l'impédance acoustique entre le premier foyer (16) et la partie du corps du patient voisine du second foyer (21) et le transfert des ondes de choc du premier (16) au second foyer (21).

8. Dispositif selon la revendication 1, et destiné à être utilisé pour la destruction, par voie non invasive, d'objets nuisibles situés dans un organisme vivant, caractérisé en ce que le guide d'onde comprend des moyens (27, 28) pour concentrer sur un premier foyer (39) pratiquement linéaire, de forme circulaire, les ondes de choc produites à son interface d'entrée (9a), et un réflecteur (19a) dont la surface est celle engendrée par la rotation d'une ellipse autour d'un axe (X) du dispositif passant par le centre dudit cercle (39) et dont un foyer suit ledit cercle (39), tandis que son autre foyer (21a) reste constamment en un même point (21) dudit axe (X), et en ce qu'un déflecteur (26) conique est disposé à proximité dudit foyer linéaire circulaire (39), pour envoyer sur le réflecteur (19a) la partie des ondes de choc provenant de ce foyer circulaire qui, en son absence, n'atteindraient pas ce réflecteur (19a), pour assurer pratiquement le transfert de toutes les ondes de choc du premier foyer (39) au second (21), un liquide étant disposé entre le réflecteur (19) et la partie du corps du patient voisine de l'objet à détruire, lorsque le second foyer (21) a été amené à l'intérieur de cet objet.

9. Dispositif selon la revendication 8, caractérisé par le fait que lesdites ondes de choc sont guidées, à travers un premier tronçon (4a) dudit guide d'onde, entre des mâchoires circulaires (27, 28), coaxiales, distantes de moins d'une longueur d'onde mesurée dans ledit liquide, constituées par la face de sortie (28) dudit tronçon guide d'onde (4a, 4c) et une masse réactive (24), de façon à engendrer, dans le liquide compris entre lesdites mâchoires (27, 28), des ondes centrifuges qui, à partir d'un cercle géométrique formant le premier foyer (29), extracorporel et situé à proximité du bord externe desdites mâchoires, rayonnent dans le volume liquide ouvert devant elles, jusqu'au second foyer (21a), intracorporel, où elles se concentrent.

10. Dispositif selon la revendication 9 et dans lequel le rayon du cercle géométrique formant le lieu du premier foyer (39) extracorporel est assez petit pour être négligé, caractérisé en ce que la surface active du réflecteur (19a) a la forme d'un ellipsoïde.


**Patentansprüche**

1. Vorrichtung zur Einwirkung auf ein Objekt mittels Ultraschall-Schwingungen, mit einem Ultraschall-Stoßwellen-Generator (1, 2, 3), dadurch **gekennzeichnet**, daß sie einerseits ein Projektil länglicher Form umfaßt, das in eine ein Blasrohr (2) bildende Röhre eingesetzt ist, um darin zu gleiten, und pneumatische Mittel (5), die an dem einen Ende des Blasrohres (2) angeordnet sind, um dieses Projektil (1) mit einer hin- und hergehenden Bewegung in dem Blasrohr (2) zu beaufschlagen mit einer Amplitude, die wesentlich größer ist als die Querabmessung des Projektils, und andererseits einen Wellenleiter (4, 19), der eine Eingangsgrenzfläche (9) aufweist, die an dem anderen Ende des Blasrohres (2) angeordnet und dafür vorgesehen ist, von dem Projektil (1) im Verlauf seiner abwechselnden Bewegung periodisch getroffen zu werden und so durch eine ballistische Wirkung Ultraschall-Stoßwellen zu erzeugen, wobei dieser Wellenleiter (4, 19) für eine Vermittlung dieser Stoßwellen an ihren Gebrauchsort (21) angeordnet ist.

2. Vorrichtung nach Anspruch 1, dadurch **gekennzeichnet**, daß der in Bezug auf das Projektil (1) stromaufwärts gelegene Teil des Blasrohres (2) direkt dem zyklischen Druck eines Verdichterzylinders ausgesetzt ist, wobei das Projektil in der Vorwärtsrichtung während der Hochdruckphase und in der Rückwärtsrichtung während der Niedrigdruckphase zirkuliert.

3. Vorrichtung nach Anspruch 1 oder 2, dadurch **gekennzeichnet**, daß das Blasrohr zu seinem stromabwärts gelegenen Ende hin mit einem Hilfsspeicher (8) pneumatisch verbunden ist, damit in diesem angesammelte Luft die Rückkehr des Projektils (1) in Richtung zu seiner Startposition hin versichert.

4. Vorrichtung nach Anspruch 2 oder 3, dadurch **gekennzeichnet**, daß der Verdichter Reguliermittel aufweist, welche die Einstellung der Luftmenge in dem Antriebskreislauf ermöglichen und als Folge davon der Amplitude der Stoßwellen.

5. Vorrichtung nach Anspruch 1, dadurch **gekennzeichnet**, daß das Projektil (1) durch eine aufeinanderfolgende Versorgung aus einer Druckluftquelle mit einem praktisch konstanten Druck angetrieben wird.

6. Vorrichtung nach Anspruch 1, die zur Verwendung als ein Lithotripter vorgesehen ist, dadurch **gekennzeichnet**, daß der Wellenleiter ein Stab (4) mit einem Durchmesser und einer Länge ist, die kompatibel mit seiner Hindurchführung durch ein Gerät des Renoscop-Typs sind und auf den Stein durch ein Abschaben bei einer Berührung einwirken.

7. Vorrichtung nach Anspruch 1, die für die Zerstörung durch nichtinvasive Mittel von schmerzvollen Objekten vorgesehen ist, welche sich in einem lebenden Organismus befinden, dadurch **gekennzeichnet**, daß der Wellenleiter einen elliptischen Reflektor (19) mit einem exkorporalen Brennpunkt (16) umfaßt, von welchem das Projektil (1) kommt, um die Oberfläche einer Flüssigkeit (18) durch eine feine Dichtungsmembrane (17) hoher Festigkeit hindurch zu beaufschlagen und damit Stoßwellen zu erzeugen, wobei der andere Brennpunkt des Reflektors (19) an den Ort gebracht werden kann, an welchem sich das zu zerstörende Objekt befindet, und wobei die Flüssigkeit (18) die Kontinuität der akustischen Impedanz zwischen dem ersten Brennpunkt (16) und dem Teil des Körpers des Patienten nahe dem zweiten Brennpunkt (21) versichert sowie die Vermittlung der Stoßwellen von dem ersten (16) zu dem zweiten Brennpunkt (21).

8. Vorrichtung nach Anspruch 1, die für die Zerstörung durch nichtinvasive Mittel von schmerzvollen Objekten vorgesehen ist, welche sich in einem lebenden Organismus befinden, dadurch **gekennzeichnet**, daß der Wellenleiter Mittel (27, 28) für eine Konzentrierung der an seiner Eingangsgrenzfläche (9a) erzeugten Stoßwellen auf einen praktisch linearen ersten Brennpunkt (39) kreisförmiger Form umfaßt und einen Reflektor (19a), dessen Oberfläche diejenige ist, die durch Drehung einer Elipse um eine Achse (X) der Vorrichtung erhalten ist, welche durch die Mitte des Kreises (39) hindurchgeht, wobei der eine Brennpunkt dem Kreis (39) folgt und der andere Brennpunkt (21a) ständig an einem gleichen Punkt (21) dieser Achse (X) verbleibt, und daß ein konischer Deflektor (26) in der Nähe des linearen kreisförmigen Brennpunktes (39) angeordnet ist, um an den Reflektor (19a) den Teil der Stoßwellen weiterzugeben, die ihren Ursprung von diesem kreisförmigen Brennpunkt haben und bei seinem Fehlen diesen Reflektor (19a) nicht erreichen würden, um dadurch praktisch die Übermittlung aller Stoßwellen von dem ersten Brennpunkt (39) zu dem zweiten (21a) zu versichern, wobei eine Flüssigkeit zwischen dem Reflektor (19) und dem Teil des Körpers des Patienten nahe dem zu zerstörenden Objekt angeordnet ist, wenn der zweite Brennpunkt (21a) in das Innere des Objekts gebracht worden ist.

9. Vorrichtung nach Anspruch 8, dadurch **gekennzeichnet**, daß die Stoßwellen über einen ersten Abschnitt (4a) des Wellenleiters zwischen kreisförmigen koaxialen Backen (27, 28) geleitet sind, die, gemessen in der Flüssigkeit, voneinander um weniger als eine Wellenlänge getrennt und durch die Austrittsfläche (28) des Wellenleiter-Abschnittes (4a, 4c) und eine reaktive Masse (24) derart ausgebildet sind, daß in der Flüssigkeit, die zwischen den Backen (27, 28) vorhanden ist, Zentrifugalwellen erzeugt werden, welche von einem geometrischen Kreis herkommen, der den ersten Brennpunkt (39) bildet, welcher extrakorporal und in der Nähe des Außenrandes der Backen angeordnet ist, wobei die Wellen in das vor ihnen offene Flüssigkeitsvolumen ausstrahlen bis hin zu dem zweiten Brennpunkt (21a), der intrakorporal ist und in welchem sie sich konzentrieren.

10. Vorrichtung nach Anspruch 9, bei welcher der Radius des geometrischen Kreises, der den Ort des extrakorporalen ersten Brennpunktes (39) bildet, genügend klein ist, um unberücksichtigt zu bleiben, dadurch **gekennzeichnet**, daß die aktive Oberfläche des Reflektors (19a) die Form eines Ellipsoides aufweist.

## Claims

1. A device for acting by ultrasonic vibrations on an object, comprising an ultrasonic shock wave generator (1, 2, 3) characterised in that it comprises, on the one hand, a projectile of elongate shape placed in a tube forming a blowpipe (2), to slide therein, and pneumatic means (5) disposed at one end of the blowpipe (2), to impart to this projectile (1) a to and fro movement in the blowpipe (2), of amplitude considerably greater than the transverse dimension of the projectile, and, on the other hand, a wave-guide (4, 19) exhibiting an entrance interface (9) situated at the other end of the blowpipe (2), and provided in order to be struck periodically by the projectile (1) in the course of its alternating movement and thus to generate, by ballistic effect, ultrasonic shock waves, this waveguide (4, 19) being arranged to transmit these shock waves to their place of utilization (21).

2. The device as claimed in claim 1, characterised in that the part upstream of said blowpipe (2), in relation to said projectile (1), is directly subjected to the cyclic pressure of a compressor cylinder, said projectile circulating in the forward sense during the high-pressure phase, and in the return sense during the low-pressure phase.

3. The device as claimed in claim 1 or 2, characterised in that said blowpipe is pneumatically connected, toward its downstream end, to an auxiliary reservoir (8), in order that air accumulated in the latter ensures the return of said projectile (1) towards its starting position.

4. The device as claimed in claim 2 or claim 3, characterised in that said compressor comprises regulable means for permitting the adjustment of the quantity of air in the propulsion circuit and, in consequence, of the amplitude of the shock waves.

5. The device as claimed in claim 1, characterised in that the projectile (1) is propelled by a sequential supply to the blowpipe (2) from a source of compressed air of a practically constant pressure.

6. The device as claimed in claim 1 and intended to be used as an lithotriptor, characterised in that the waveguide is a rod (4), of diameter and length which are compatible with its passage through an apparatus of the renonscope type and operating on the stone by contact chipping.

7. The device as claimed in claim 1 and intended to be used for the destruction, by non-invasive means, of harmful objects, which are situated in a living organism, characterised in that the waveguide comprises an ellipsoidal reflector (19), with extracorporal focus (16) from which the projectile (1) comes to strike the surface of a liquid (18), via a fine sealing membrane with high strength (17), to produce shock waves thereat, the other focus of the reflector (19) being capable of being brought to the place where the object to be destroyed is situated, the liquid (18) ensuring the continuity of the acoustic impedance between the first focus (16) and the part of the body of the patient close to the second focus (21) and the transfer of the shock waves from the first (16) to the second focus (21).

8. The device as claimed in claim 1, and intended to be used for the destruction, by non-invasive means, of harmful objects situated in a living organism, characterised in that the waveguide comprises means (27, 28) for concentrating on a practically linear first focus (39), of circular shape, the shock waves produced at its entrance interface (9a), and a reflector (19a), the surface of which is that created by the rotation of an ellipse about an axis (X) of the device passing through the center of said circle (39) and a focus of which follows said circle (39), while its other focus (21a) remains constantly at a same point (21) of said axis (X), and that a conical deflector (26) is disposed in proximity to said circular linear focus (39), to pass to the reflector (19a) the part of the shock waves originating from this circular focus which, in its absence, would not reach this reflector (19a), practically to ensure thereby the transfer of all the shock waves from the first focus (39) to the second (21a), a liquid being disposed between the reflector (19) and the part of the body of the patient close to the object to be destroyed, when the second focus (21a) has been brought into the interiour of this object.

9. The device as claimed in claim 8, characterised in that said shock waves are guided, via a first section (4a) of said waveguide, between circular coaxial jaws (27, 28), separated by less than one wavelength measured in said liquid, constituted by the exit face (28) of said waveguide section (4a, 4c) and a reactive mass (24), in such a manner as to generate, in the liquid contained between said jaws (27, 28), centrifugal waves which, proceeding from a geometric circle forming the first focus (39), which is extracorporal and situated in proximity to the external edge of said jaws, radiate into the liquid volume which is open in front of them, as far as to the second focus (21a), which is intracorporal and at which they concentrate.

10. The device as claimed in claim 9 and in which the radius of the geometric circle forming the locus of the extracorporal first focus (39) is small enough to be disregarded, characterised in that the active surface of the reflector (19a) has the shape of an ellipsoid.

FIG.1

FIG. 2

**FIG. 3**

**FIG. 4**

EP 0 317 507 B1